# EUROPEAN PATENT SPECIFICATION

(11) **EP 3 829 499 B1**
(45) Date of publication and mention of the grant of the patent: **04.12.2024**
(21) Application number: 19730881.0
(22) Date of filing: 28.05.2019
(51) Int. Cl.: A61F 7/02, A61F 7/10

(54) **A NOSE CLAMP**
NASENKLAMMER
PINCE-NEZ

(30) Priority: 31.07.2018 GB 201812519
(43) Date of publication of application: 09.06.2021
(73) Proprietor: Minks, Wendy, Bristol BS30 5TX (GB)
(72) Inventor: Minks, Wendy, Bristol BS30 5TX (GB)
(74) Representative: Definition IP Limited
(86) International application number: PCT/GB2019/051453
(87) International publication number: WO 2020/025917

(56) References cited:
- WO-A1-2014/080222
- CN-A- 1 552 483
- DE-U1- 202013 009 004
- US-A1- 2009 299 405

## Description

### FIELD OF THE INVENTION

The present invention relates to a nose clamp. In particular, the present invention relates to a nose clamp to provide a compressing and temperature altering effect on a nose.

### BACKGROUND OF THE INVENTION

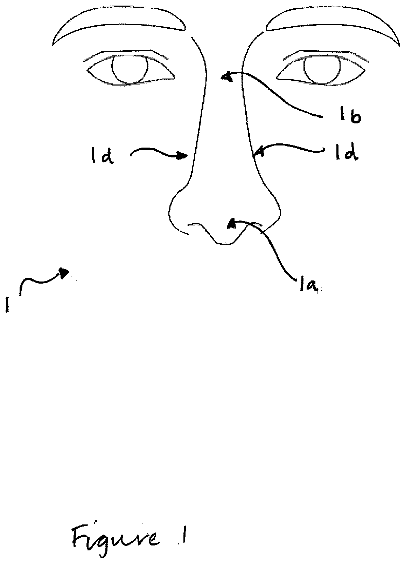

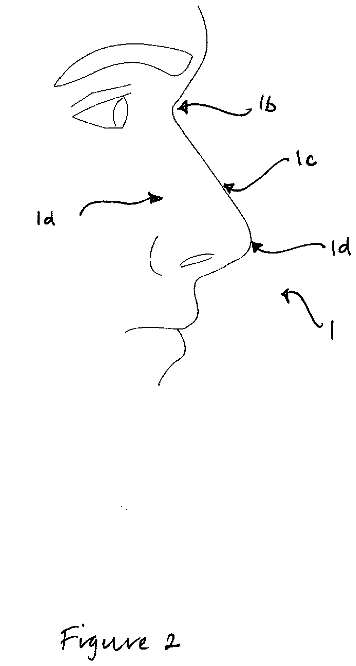

Referring to Figures 1 and 2, a human nose 1 is shown. The human nose 1 comprises a tip (1a) located at the end of the nose and a bridge (1b) at the top of the nose between the eye sockets. A dorsum (1c) extends between the tip and the bridge. Nasal sidewalls (1d) are cartilaginous portions arranged on either side of the dorsum, anterior to the nasal bone.

It is known that compression, cooling and/or heating can provide a supporting and soothing effect to an injured nose. The compression, cooling and/or heating can also help to control inflammation and swelling of the injured nose.

Nosebleeds typically occur in an anteroinferior part of the nasal septum, medial to the nasal sidewalls (sometimes referred to as the Kiesselbach's plexus or in Little's area). It is commonly advised to apply a pinching pressure to the nose to stem the blood flow. There is also evidence that cooling and/or heating can help to promote the clotting of the blood.

Additionally, it has been found that compression, cooling and/or heating can benefit noses intra-operatively or post-operatively so as to control inflammation and swelling.

Devices may be utilised to compress an injured nose. However, current nose compressing devices are typically complex and comprise multiple parts. They are not self-holding and include complicated closure mechanisms that require manual adjustment to fit the nose. These manually adjustable closure mechanisms allow for user error and can lead to under tightening or over-tightening. The closure mechanisms can also increase the overall size, weight and cost of the devices. It has been found that the compressing effect provided by some devices is limited and has little beneficial effect. Certain other devices provide a pointed compressing effect over a limited surface area of the nose, and this prolonged point compression can lead to point necrosis damaging tissues in the nose. DE202013009004U1 and WO2014/080222 disclose nose clamps of the prior art.

### SUMMARY OF THE INVENTION

The present invention seeks to provide an alternative and improved nose clamp and method of mounting a nose clamp. The present invention also seeks to address the problems mentioned above.

The invention is defined in appended independent claims 1 and 14, further embodiments are described in the dependent claims.

A first aspect of the present invention relates to a nose clamp. The nose clamp comprises a resilient clamping body and a temperature altering body, wherein the resilient clamping body is configured to apply a compressing force so as to compress a user's nose and urge the temperature altering body into contact with the nose.

The resilient clamping body comprises a bridge extending between a first jaw and a second jaw, wherein each jaw comprises an outer wall, an inner wall and a folding joint between the outer wall and the inner wall such that the inner wall folds inwardly from a lower end of the outer wall. In use, the clamping body seats the nose clamp on the nasal sidewalls of the user's nose, anterior to the nasal bone. The bridge is configured to extend over the dorsum of a user's nose, and the jaws are configured to extend around the nasal sidewalls of the user's nose and engage with the temperature altering body.

The clamping body is resilient so as to allow for deformation from its original shape during the mounting on the user's nose and urge the nose clamp back into its original shape after deformation. Therefore, when mounted on a nose, the deformed jaws exert a compressing force, that has a clamping effect on the nasal sidewalls, as they seek to spring back to their original position.

The compressing force provided by the jaws is able to hold the temperature altering body in abutting contact against the nasal sidewalls so that the temperature altering body can provide a cooling and/or heating effect on the user's nose. The compressing force provided by the jaws is also sufficient to control (preferably minimise) inflammation and swelling of the user's nose and provide support. If the nose is bleeding, the compressing force acting on the nasal sidewalls is able to compress ruptured blood vessels and encourage clotting.

By using resilient jaws, the nose clamp is easy to operate, it is self-holding and self-adjusting to a user's nose. No manual adjustment mechanisms are necessary and user error is mitigated.

The inner walls of the jaws are inwardly folded from the lower end of the respective outer walls by the folding joints such that they extend in a return direction towards the bridge on the inner sides of the respective outer walls. The double walled jaw configuration provides additional resilience to enhance the compressing force of the clamping body. The folding joints act as resilient joints that allow the inner walls to flex independently of the respective outer walls, and this further augments the clamping effect.

Due to the double walled jaw configuration, the compressing force of the clamping body acts via the inner walls. The inner walls of the jaws are preferably configured to correspond to the shape of the respective nasal sidewalls. As a consequence, the fit of the nose clamp is improved and is more comfortable to wear for the user. Also, the compressing force of the clamping body is exerted via the inner wall more evenly and applied substantially across all the surface area of the nasal sidewalls of the user's nose. Hence, the risk of point necrosis is avoided. Additionally, the inner walls are able to urge the temperature altering body into improved abutting contact with the nasal sidewalls to achieve a more even cooling and/or heating effect and substantially across all the surface area of the nasal sidewalls.

In an embodiment, the resilient clamping body may be a one-piece body. As a one-piece clamping body, the bridge and jaws are integrally formed. As a result, the clamping body is a simple and compact design, and it can be manufactured easily with minimal waste and reduced weight in comparison to known nose clamps.

The clamping body may be made of either nylon or polypropylene, or any suitable resilient material.

The bridge may comprise a central region, a first haunch region and a second haunch region.

The first haunch region and second haunch region may curve inwardly from the central region. As a consequence, the rigidity and tension of the clamping body is improved, which in turn aids the compressing effect of the nose clamp.

In an embodiment, the clamping body may comprise at least one reinforcement ridge extending between the bridge and at least one of the jaws. The reinforcement ridge acts to increase the resilience of the clamping body and thereby improve the compressing effect of the nose clamp. The reinforcement ridge also improves the rigidity of the nose clamp to reduce stress at the joint between the bridge and the at least one jaw. As a consequence, the nose clamp can undergo increased operational cycles and so the life of the nose clamp is prolonged.

The bridge may comprise a front edge located proximal the tip of the user's nose in use, and a rear edge located distal the tip of the user's nose in use. Likewise, the outer and inner walls of each of the jaws may each comprise a front edge located proximal the tip of a nose in use and a rear edge located distal the tip of the user's nose in use.

In an embodiment, the front edge of the bridge may comprise a first recess and/or the rear edge of the bridge may comprise a second recess. The rear edges of the outer walls of the jaws may each comprise a recess. The recesses in the bridge and/or jaws reduce the overall weight of the clamping body. The recesses also help to improve the fit of the bridge or jaws by providing a more ergonomic design and avoid contact between the nose clamp and the user's nasal bone.

The front edges of the inner and outer walls of each of the jaws may be substantially perpendicular and/or flush.

The rear edges of the outer walls of the jaws may be inclined and/or curved. Preferably, the inclination and/or curving of the rear edges of the outer walls follow the contours of the user's face, therefore improving user comfort and avoiding contact between the nose clamp and the user's nasal bone.

At least a portion of the rear edges of the inner walls of the jaws may be inclined or inwardly curved. Preferably the inclined or inwardly curved portions follow the contours of the nasal sidewalls and further contribute to user comfort by avoiding contact between the nose clamp and the user's nasal bone.

The rear edge of the inner walls may be recessed from the rear edge of the outer walls. Advantageously, this provides a more ergonomic design, allowing the shape of the inner wall to follow the contours of the nasal sidewalls of the nose.

The temperature altering body is configured to provide a cooling, heating or an alternate cooling and heating effect on the nose, as desired.

In use, the temperature altering body is engaged by one or both inner walls of the jaws for placement against one or both of the nasal sidewalls. The cooling and/or heating effect on the nasal sidewalls of the nose can provide a soothing effect, control inflammation and swelling, and promote the clotting of blood from any ruptured blood vessels in the user's nose to help stop a nosebleed.

The temperature altering body may be separate from the clamping body and mountable on the user's nose such that it extends over the nasal sidewalls and is urged against the nose by the clamping body. The temperature altering body may be fixable to one or both jaws of the clamping body. Alternatively, the temperature altering body may be integrally formed with the jaws of the clamping body.

The temperature altering body may be a one-piece body fixable to the inner wall of one or both jaws. Alternatively, the temperature altering body may comprise a first pad fixable to the first inner wall and a second pad fixable to the second inner wall.

Also disclosed but not claimed is a method of clamping a user's nose using the nose clamp according to the first aspect of the invention. The method comprises:
opening the jaws of the clamping body;
positioning the bridge over the dorsum of the user's nose and locating the inner walls of the jaws adjacent to respective nasal sidewalls;
releasing the jaws to exert a compressive force via the inner walls, the compressive force compressing the nasal sidewalls and urging the temperature altering device in abutting contact with the nasal sidewalls.

A further aspect of the invention relates to a kit of parts for a nose clamp, the kit comprising a clamping body and a temperature altering body according to the first aspect of the invention.

### BRIEF DESCRIPTION OF THE DRAWINGS

For a better understanding of the invention and to show how it may be carried into effect reference shall now be made, by way of example only, to the accompanying drawings, in which:
Figure 1 shows a front view of a human nose;
Figure 2 shows a side view of a human nose;
Figure 3 shows a front perspective view of a first embodiment of a clamping body of a nose clamp in accordance with the present invention;
Figure 4 shows a front view of the clamping body of the nose clamp of Figure 3;
Figure 5 is a side view of the clamping body of the nose clamp of Figure 3;
Figure 6 is a rear perspective view of the clamping body of the nose clamp of Figure 3;
Figure 7 is a top view of the clamping body of the nose clamp of Figure 3;
Figure 8 is a cross-section view of the clamping body of the nose clamp of Figure 3;
Figure 9 is a front perspective view of a second embodiment of a clamping body of a nose clamp in accordance with the present invention;
Figure 10 is a rear perspective view of the clamping body of the nose clamp of Figure 9;
Figure 11 is a front perspective view of a first embodiment of a temperature altering body fixed to the clamping body of the nose clamp of Figure 3;
Figure 12 is a front view of the temperature altering body fixed to the clamping body of the nose clamp of Figure 11;
Figure 13 is a front perspective view of a second embodiment of a temperature altering body fixed to the clamping body of the nose clamp of Figure 3;
Figure 14 is a front view of the temperature altering body fixed to the clamping body nose clamp of Figure 13;
Figure 15 is a side view showing the nose clamp of Figure 11 mounted on a user's nose;
Figure 16 is a front view showing the nose clamp of Figure 11 mounted on a user's nose; and
Figure 17 is a front view showing the nose clamp of Figure 13 mounted on a user's nose.

### DETAILED DESCRIPTION OF EMBODIMENT(S)

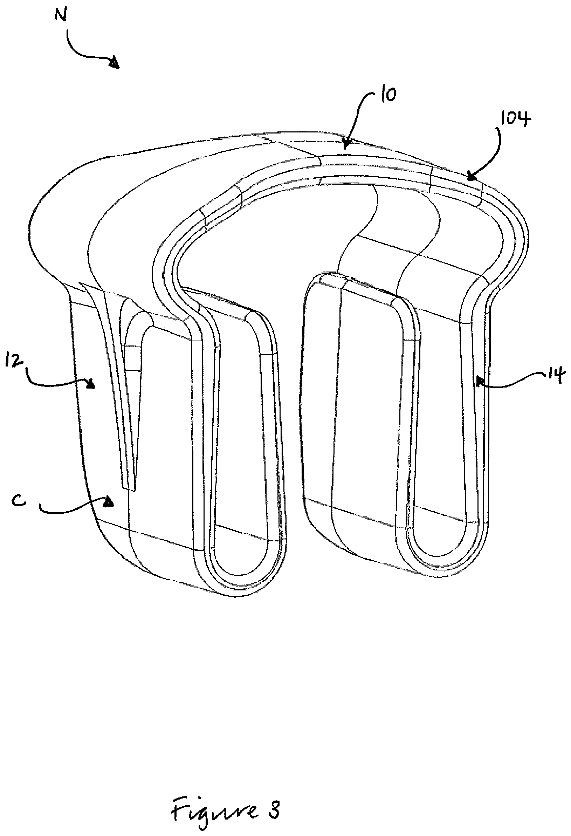

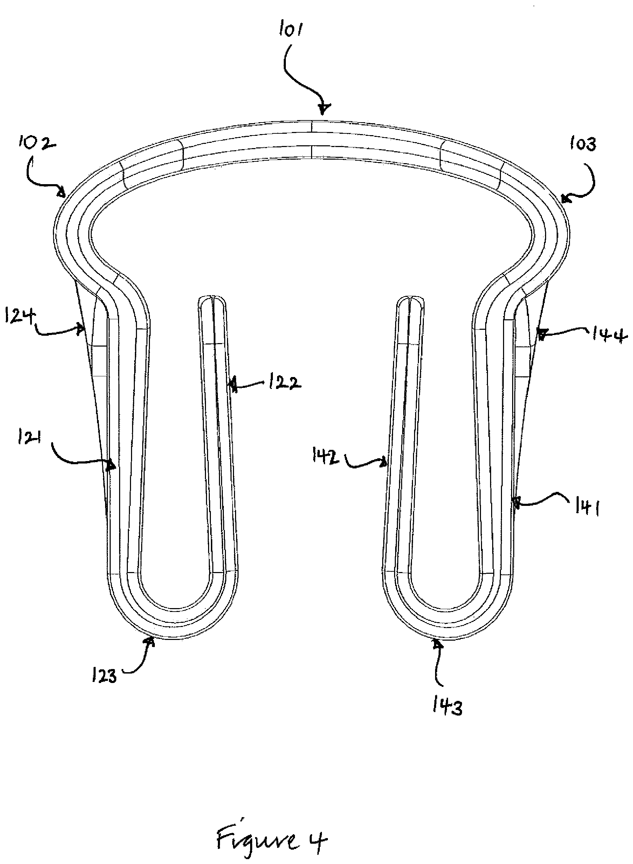

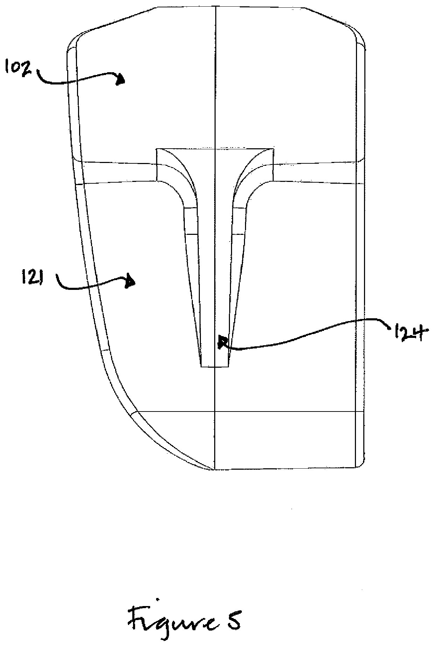

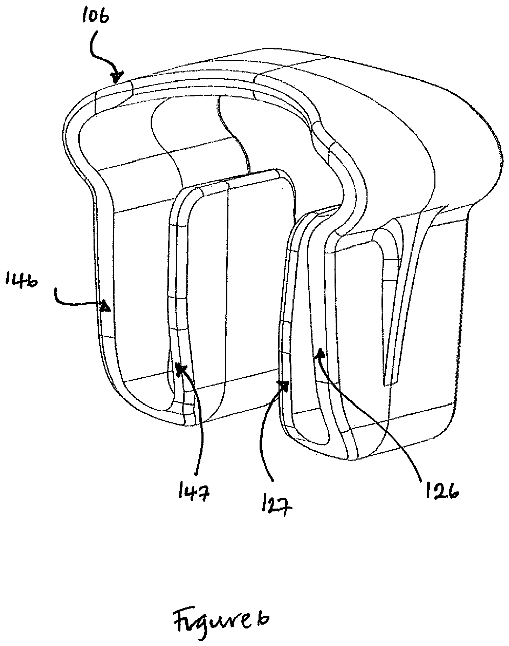

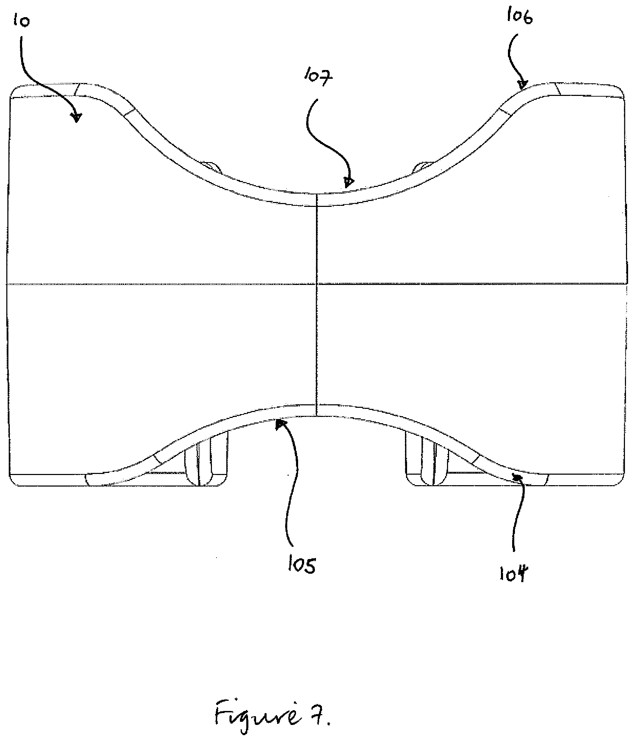

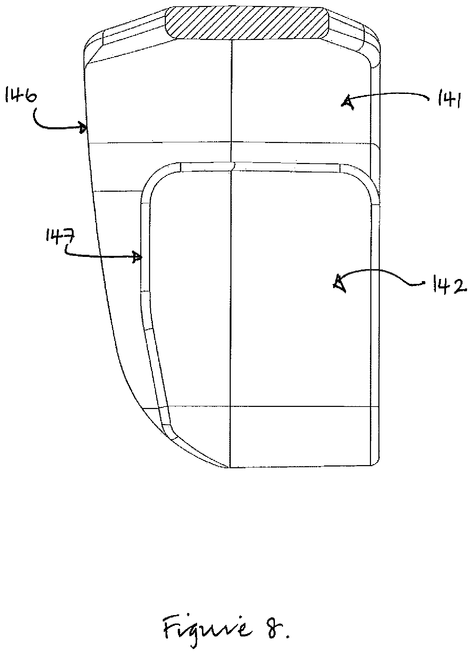

Referring now to Figures 3 to 17, a first aspect of the present invention relates to a nose clamp. The nose clamp (N) comprises a resilient clamping body (C) and a temperature altering body (T). In use, the clamping body is configured to apply a compressing force to compress the user's nose and urge the temperature altering body into contact with the nose.

The nose clamp is suitable for use with injured noses, including nosebleeds, intra-operative noses or post-operative noses.

The nose clamp may be appropriately dimensioned for use on a human or animal nose.

Figures 3 to 17 depict different embodiments of the nose clamp (N) where the resilient clamping body (C) comprises a bridge (10,10') extending between a first jaw (12,12') and a second jaw (14,14'). The bridge is configured to extend over the dorsum (1c) of the user's nose. The jaws are configured to extend around the nasal sidewalls (1d) of the nose and engage with the temperature altering device that is arranged between the clamping body and the nasal sidewalls. As such, the clamping body seats the nose clamp on the nasal sidewalls of the user's nose.

As depicted in the Figures, the resilient clamping body may be a one-piece body whereby the bridge and jaws are integrally formed. The resilient clamping body may be formed from any suitable resilient material, including for example, nylon or polypropylene.

The bridge comprises a central region, first haunch region and second haunch region.

The bridge may have an arc shape or any suitable shape to extend over the dorsum of a user's nose. The bridge may have a low or high profile arc shape. The bridge may be curved with a three centre point arc shape.

In a first embodiment of the clamping body depicted in Figures 3 to 8, the bridge (10) comprises a central region (101), first haunch region (102) and second haunch region (103) wherein the first and second haunch regions curve inwardly from the central region. The inward curve of the first and second haunch regions may range from 140° to 180°.

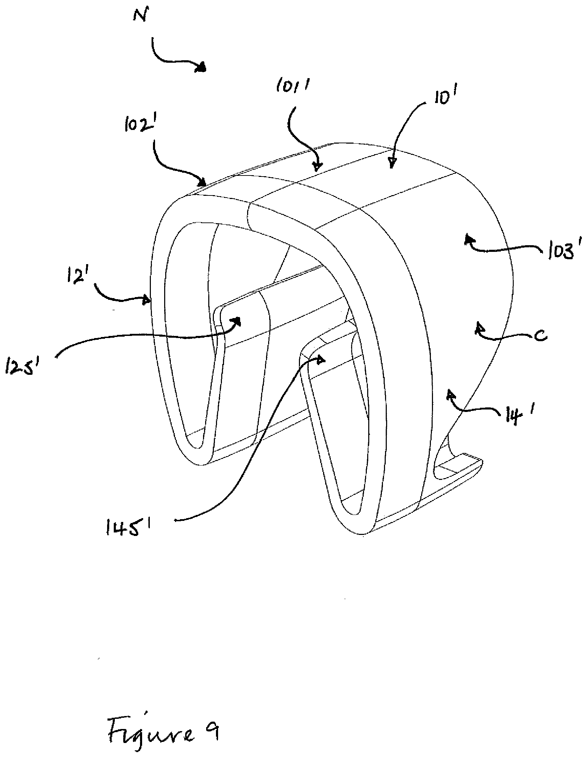

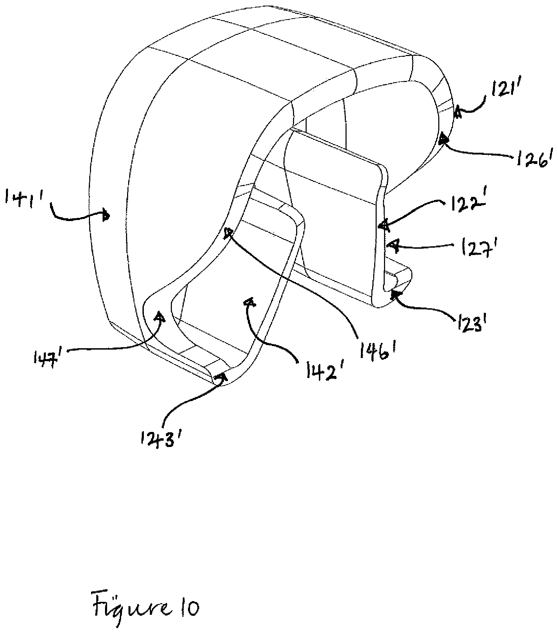

In a second embodiment of the clamping body depicted in Figures 9 & 10, the bridge (10') comprises a central region (101'), first haunch region (102') and second haunch region (103') wherein the first and second haunch regions curve downwardly from the central region. The downward curve of the first and second haunch regions may range from 60° to 90°.

To enhance the overall resilience of the clamping body and thereby the clamping effect, the jaws of the clamping body are doubl walled jaws. The first jaw comprises a first outer wall, a first inner wall and a first folding joint between the first outer wall and first inner wall. The second jaw comprises a second outer wall, a second inner wall and a second folding joint between the second outer wall and second inner wall. The folding joints are arranged between the lower ends of the outer walls and inner walls of the jaws. The inner walls are inwardly folded at the folding joints from the respective outer walls such they form a 'return wing' extending in a direction towards the bridge on the inner side of the outer walls.

As shown in the Figures, the folding joints may be a return bend in the jaw ranging from 140° to 180°. Alternatively, the folding joint may be any suitable joint to provide the folding effect between the outer walls and inner walls. The folding joints are resilient joints that allow the inner walls to flex independently of the respective outer walls.

In the first embodiment of the clamping body depicted in Figures 3 to 8, the first jaw (12) comprises a first outer wall (121), a first inner wall (122) and a first folding joint (123) between the first outer wall and first inner wall. The second jaw (14) comprises a second outer wall (141), a second inner wall (142) and a second folding joint (143) between the second outer wall and second inner wall. In the original position, the outer walls of the jaws are substantially straight and extend downwardly (generally perpendicularly) from the respective haunch regions (102, 103) of the bridge. The folding joints are 180° return bends arranged at the bottom of each jaw between the lower ends of the outer walls and inner walls. The inner walls are substantially straight. Due to the configuration of the 180° return bends of the folding joints the inner walls return from the respective outer walls and extend in a direction upwardly towards the bridge, generally parallel to the respective outer walls.

In the second embodiment of the clamping body depicted in Figures 9 & 10, the first jaw (12') comprises a first outer wall (121'), a first inner wall (122') and a first folding joint (123') between the first outer wall and first inner wall. The second jaw (14') comprises a second outer wall (141'), a second inner wall (142') and a second folding joint (143') between the second outer wall and second inner wall. In the original position, the outer walls extend downwardly from the respective haunch regions (102', 103') of the bridge with an inwardly curved profile. The folding joints are 150° return bends arranged at the bottom of each jaw between the lower ends of the outer walls and inner walls. The inner walls are substantially straight. Due to the configuration of the 150° return bends of the folding joints the inner walls return from the respective outer walls and extend upwardly towards the bridge, at an incline away from the inner side of the respective outer walls.

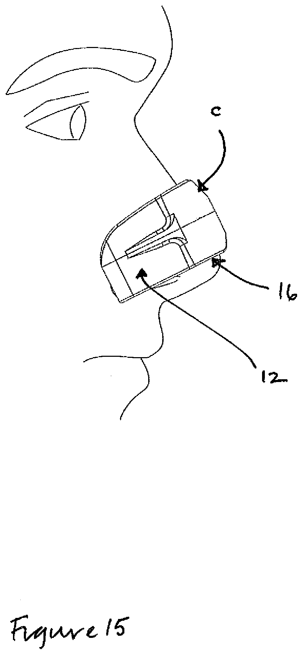

Due to the double walled jaws, the compressing force of the clamping body acts via the inner walls to compress the nasal sidewalls and urge the temperature altering body into contact with the nasal sidewalls. The inner walls are preferably configured to correspond to the shape of the nasal sidewalls so as to optimise indirect contact of the inner walls on the nasal sidewalls and direct contact of the temperature altering body on the nasal sidewalls. As a result, the inner walls are able to conform to the contours of the nasal sidewalls so that they are able to more evenly apply a compressing force over substantially all the surface area of the nasal sidewalls when the nose clamp is in use. Also, the inner walls enhance the abutting contact between the temperature altering body and the nasal sidewalls so that the cooling and/or heating effect is provided more evenly and substantially across all the surface area of the nasal sidewalls. See Figures 15 to 17.

To augment the resilience of the clamping body, and thereby the compressing effect and lifespan of the nose clamp, the clamping body may comprise one or more reinforcement ridge. In the first embodiment of the clamping body depicted in Figures 3 to 8, the clamping body comprises a first reinforcement ridge (124) extending from the first haunch region (102) of the bridge along at least part of the first outer wall (121) of the first jaw and a second reinforcement ridge (144) extending from the second haunch region (103) of the bridge along at least part of the second outer wall (141) of the second jaw.

The inner walls of the jaws may comprise lips located distal from the respective folding joints. The lips are configured to aid the mounting of the nose clamp and user comfort by avoiding inner wall contact with the dorsum of the nose. As shown in the second embodiment of the clamping body depicted in Figures 9 & 10, the first inner wall (122') comprises a lip (125') and the second inner wall (142') comprises a lip (145'). These lips are located distal from the respective folding joints and bend towards the respective outer walls.

The bridge of the clamping body comprises a front edge located proximal the tip of the user's nose in use, and a rear edge located distal the tip of the user's nose in use. Likewise, the walls of each jaw each comprise a front edge located proximal the tip of the user's nose in use and a rear edge located distal the tip of the users nose in use.

One or more edges of the bridge and/or walls of the jaws may comprise a recesses to aid the fit of the clamping body on the user's nose and reduce the weight of the clamping body.

In the first embodiment of the clamping body depicted in Figures 3 to 8, the bridge (10) comprises a front edge (104) with a front recess (105) and a rear edge (106) with a rear recess (107). The front recess and rear recess are generally curved.

The front edges of the outer and inner walls of the jaws are preferably flush as shown in the embodiments depicted in Figures 3 to 10.

The rear edge of the outer walls and/or inner walls of the jaws may be inclined, curved and/or recessed to follow the contours of the user's face and nose, thereby improving the fit and comfort of the nose clamp.

In the first embodiment of the clamping body depicted in Figures 3 to 8, the first outer wall (121) comprises a rear edge (126), the first inner wall (122) comprises a rear edge (127), the second outer wall (141) comprises a rear edge (146) and the second inner wall comprises a rear edge (147). The rear edges of the outer walls are inclined such that they taper towards the lower end of the outer wall. The rear edges at the lower ends of both the outer wall and inner walls are radiused. The rear edge of the inner walls are recessed from the rear edge of the respective outer walls so as to follow the shape of the nasal sidewalls, thereby contributing towards user comfort and the clamping effect.

In the embodiment depicted in Figures 9 & 10, the first outer wall (121') comprises a rear edge (126') with a rear recess (127') and the second outer wall (141') comprises a rear edge (146') with a rear recess (147'). The recesses are mirror recesses and generally C shaped.

The nose clamp comprises a temperature altering body to provide a temperature altering effect on the nose. The temperature altering body may be selected to provide a cooling effect, heating effect or an alternate cooling and heating effect. Temperature altering technology for providing a cooling and/or heating effect is well known. For example, the temperature altering body may comprise hydrocolloid or any other suitable cooling gel material to provide a cooling effect. The temperature altering body may provide a heating effect using an exothermic oxidation or crystallisation. The temperature altering body may comprise a Peltier device to provide an alternating cooling and heating effect.

The temperature altering body may be disposable or reusable. The temperature altering body may be manually activated, for example by squeezing or shaking. The temperature altering body may be activated by the compressing force of the clamping body as the temperature altering body is urged into abutting contact with the nose.

The temperature altering body may a one piece elongate body separately mountable on the user's nose from the clamping body. This type of body may be configured to be initially placed over a user's nose and extend along the nasal sidewalls. The clamping body is then mounted to sit on the nasal sidewalls of the user's nose over the temperature altering body so as to urge the temperature altering body into abutting contact with the nasal sidewalls and provide a temperature altering effect.

The temperature altering body may be a one piece elongate body fixable to both the inner walls of the clamping body.

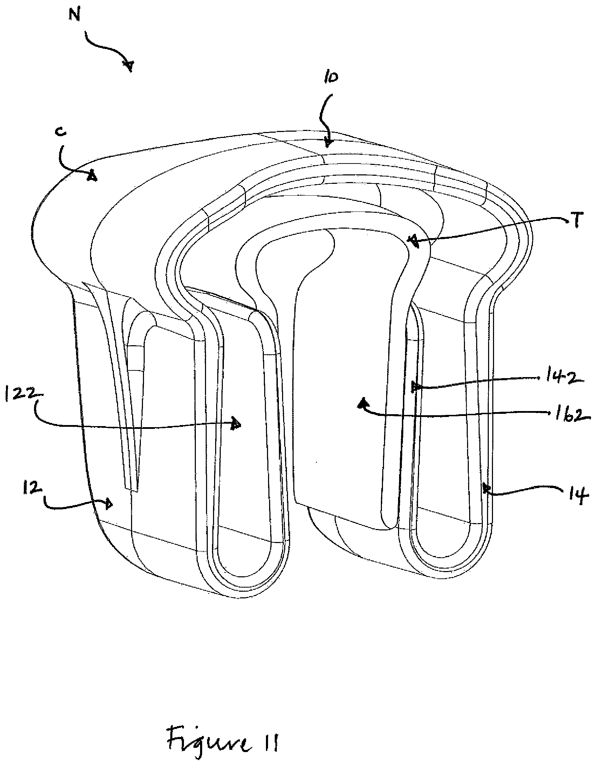

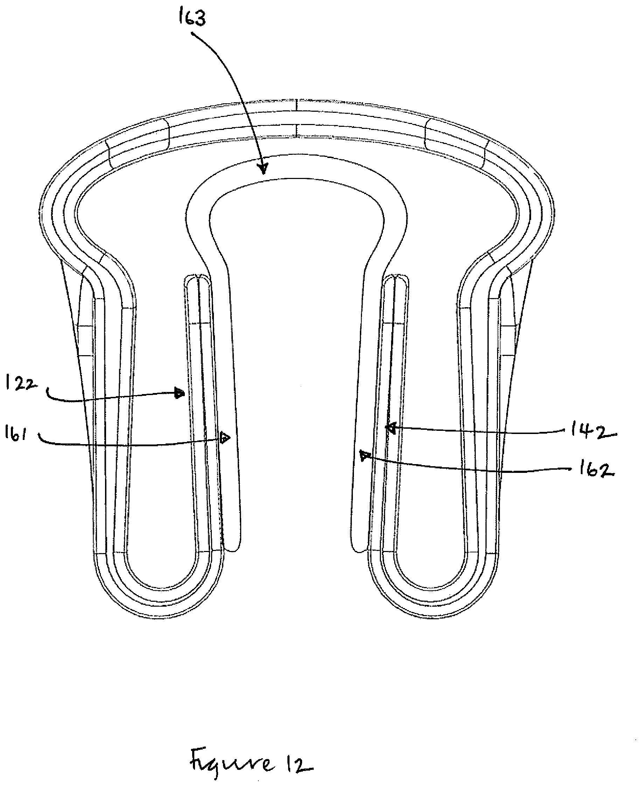

In the embodiment depicted in Figures 11 & 12, the temperature altering body is a one piece elongate cooling pack (16) comprising menthol infused hydrocolloid on a mesh backing. The cooling pack comprises a first portion (161) coupled to an inner surface of the first inner wall (122) of the first jaw, a second portion (162) coupled to an inner surface of the second inner wall (142) of the second jaw, and a third portion (163) forming a central loop between the first portion and second portion.

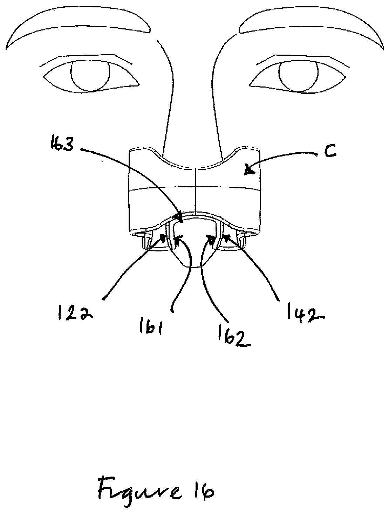

The first and second portions are configured to correspond to the shape of the inner walls, and thereby the shape of the nasal sidewalls. Hence, when the nose clamp is seated on the nasal sidewalls as shown in Figures 15 & 16 and the compressing force of the inner walls of the clamping body urge the cooling pack towards the nose, contact with the nasal sidewalls is optimised and an even cooling effect substantially across the surface area of the nasal sidewalls is achieved.

The first and second portions may be permanently adhered to the inner walls using adhesive or any other suitable permanent fixing. Alternatively, the first and second portion may be removably attached to the inner walls using any suitable removable fixing.

The temperature altering body may comprise two pieces fixable to the respective inner walls of the clamping body.

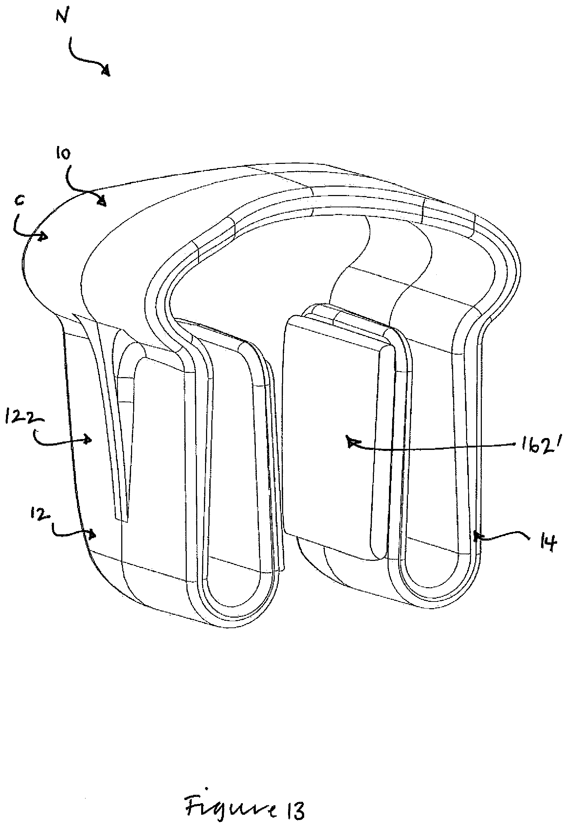

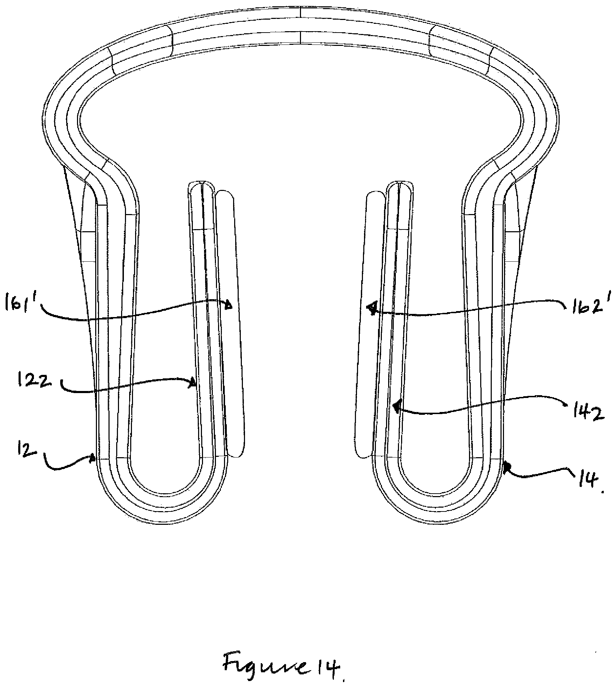

In the embodiment depicted in Figures 13 & 14, the temperature altering body is a two piece cooling pack (16') comprising a first cooling member (161') coupled to the inner surface of the first inner wall (122) of the first jaw, a second cooling member (162') coupled to the inner surface of the second inner wall (142) of the second jaw.

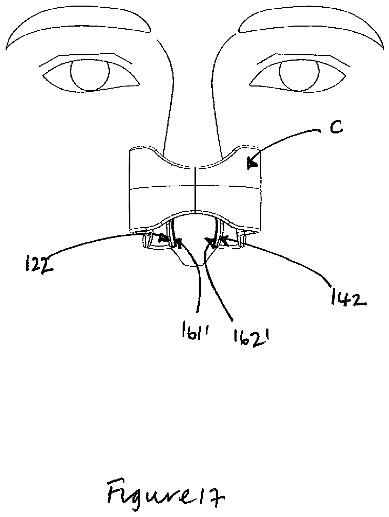

The first and second cooling members (161', 162') are configured to correspond to the shape of the inner walls, and thereby the shape of the nasal sidewalls. Therefore, when the nose clamp is seated on the nasal sidewalls of the user's nose and the compressing force of the inner walls of the clamping body urge the cooling pack members towards the nose, contact with the nasal sidewalls is optimised and an even cooling effect substantially across the surface area of the nasal sidewalls is achieved. See Figures 17.

The first and second cooling members may be permanently adhered or removably attached to the inner walls.

Alternatively, the temperature altering body may be integrally formed in the inner walls of the clamping body. For example, the inner walls may comprise a cooling material, heating material, or a Peltier device to achieve the desired effect.

Also disclosed is a method of mounting the nose clamp on a user's nose.

In order to mount the nose clamp onto a user's nose, the first jaw and the second jaw of the clamping body are separated further apart, the clamping body is then positioned over the user's nose such that the bridge extends over the dorsum of the user's nose and the first inner wall and the second inner wall are located adjacent the nasal sidewalls of the user's nose. The jaws are then released such that the first inner wall (122, 122') and the second inner wall (142, 142') exerts a compressive force compressing the nasal sidewalls 1d of the user's nose and urging the temperature altering body against the nasal sidewalls. The clamping body exerts a compressive force on the nasal sidewalls and temperature altering device by virtue of the resilience of the clamping body.

The clamping body is configured to apply a compressing force that is sufficient to provide a supporting effect, control inflammation and swelling, and when necessary, compress blood vessels to stem a nosebleed.

The cooling and/or heating effect provided by the temperature altering body on the nasal sidewalls of the nose also provides a soothing effect, and can control inflammation and swelling. The cooling and/or heating has been found to cause vasoconstriction which increases the rate at which blood clots and help reduce the time for a nosebleed to stop.

If the temperature altering body is fixable to the clamping body, then the method further comprises fixing the temperature altering body to the first and second inner walls of the jaws. Alternatively, if the temperature altering body is separate from the clamping body, then the method further comprises arranging the temperature altering body over the user nose and along the nasal sidewalls prior to seating the clamping body on the nasal sidewalls.

A further aspect of the present invention relates to a kit of part to form the nose clamp. The kit comprises the clamping body and the temperature altering body as described above.

Whilst endeavouring in the foregoing specification to draw attention to those features of the invention believed to be of particular importance, it should be understood that the applicant claims protection in respect of any patentable feature or combination of features referred to therein, and/or shown in the drawings, whether or not particular emphasis has been placed thereon.

Throughout the description and claims of this specification, the words "comprise" and "contain", and any variations of the words, means "including but not limited to" and is not intended to (and does not) exclude other features, elements, components, integers or steps.

Throughout the description and claims of this specification, the singular encompasses the plural unless the context requires otherwise. In particular, where the indefinite article is used, the specification is to be understood as contemplating plurality as well as singularity, unless the context requires otherwise.

Features, integers or characteristics described in conjunction with a particular aspect, embodiment or example of the invention are to be understood to be applicable to any other aspect, embodiment or example described herein unless incompatible therewith.

## Claims

1. A nose clamp comprising a resilient clamping body and a temperature altering body to provide a temperature altering effect on a user's nose, the clamping body comprising:
a bridge (10) to extend over a dorsum of a user's nose; and
a first jaw (12) and a second jaw (14) to extend from the bridge (10) along respective nasal sidewalls of the user's nose so as to apply a compressing force to compress the nasal sidewalls of a user's nose and urge the temperature altering body into contact with the nasal sidewalls;
wherein each jaw (12, 14) comprises an outer wall (121, 141), an inner wall (122, 142), and a resilient folding joint (123, 143) between the outer wall (121, 141) and the inner wall (122, 142) to fold the inner wall (122, 142) inwardly from the outer wall (121, 141);
wherein the shape of the inner wall (122, 142) of each jaw (12, 14) corresponds to the shape of the respective nasal sidewall.

2. The nose clamp according to any preceding claim, wherein the clamping body is a one-piece body.

3. The nose clamp according to any preceding claim, wherein the clamping body is formed from nylon or polypropylene.

4. The nose clamp according to any preceding claim, wherein the bridge (10) comprises a central region (101), first haunch region (102) and second haunch region (103) wherein the first haunch region (102) and second haunch region (103) curve inwardly from the central region (101).

5. The nose clamp according to any preceding claim, further comprising at least one reinforcement ridge (124) extending between the bridge (10) and at least one jaw (12, 14).

6. The nose clamp according to any preceding claim, wherein the bridge (10) comprises a proximal front edge (104) and a distal rear edge (106), wherein the front edge (104) comprises a recess (105) to avoid the tip of the user's nose and/or the rear edge (106) comprises a recess (107) to avoid the nasal bone of the user's nose.

7. The nose clamp according to any preceding claim, wherein the outer walls (121, 141) and inner walls (122, 142) of each jaw (12, 14) comprise a proximal front edge and a distal rear edge (126, 127, 146, 147), wherein the rear edges of the outer walls (126, 146) of the jaw (12, 14) are inclined or curved to follow the contours of a user's face.

8. The nose clamp according claim 7, wherein the rear edges of the inner walls (127, 147) of jaws (12, 14) are inclined, curved and/or recessed from the rear edges of the outer walls (126, 146) of the jaws (12, 14) to follow the contours of the respective nasal sidewalls.

9. The nose clamp according to any preceding claim, wherein the temperature altering device is configured to provide a cooling, heating or an alternating cooling and heating effect.

10. The nose clamp according to any preceding claim wherein temperature altering device is fixable to one or both of the inner walls (122, 142) of the jaws (12, 14) for placement against one or both of the nasal sidewalls in use.

11. The nose clamp according to any preceding claim wherein the temperature altering body comprises a one-piece body.

12. The nose clamp according to any of claims 1 to 10, wherein the temperature altering body comprises a first pad and a second pad fixable to the respective inner walls (122, 142) of the jaws (12, 14).

13. The nose clamp according to any of claims 1 to 10, wherein the temperature altering device is integrally formed in each inner wall (122, 142) of the clamping body.

14. A kit of parts to form a nose clamp as defined in any of claims 1 to 13, the kit comprising a resilient clamping body and a temperature altering body to provide a temperature altering effect on a user's nose, the clamping body comprising:
a bridge (10) to extend over a dorsum of a user's nose; and
a first jaw (12) and a second jaw (14) to extend from the bridge (10) along respective nasal sidewalls of the user's nose so as to apply a compressing force to compress the nasal sidewalls of a user's nose and urge the temperature altering body into contact with the nasal sidewalls;
wherein each jaw (12, 14) comprises an outer wall (121, 141), an inner wall (122, 142), and a resilient folding joint (123, 143) between the outer wall (121, 141) and the inner wall (122, 142) to fold the inner wall (122, 142) inwardly from the outer wall (121, 141);
wherein the shape of the inner wall (122,142) of each jaw (12,14) corresponds to the shape of the respective nasal sidewall.

## Patentansprüche

1. Nasenklemme, umfassend einen elastischen Klemmkörper und einen temperaturverändernden Körper, um eine temperaturverändernde Wirkung auf die Nase eines Benutzers bereitzustellen, der Klemmkörper umfassend:
eine Brücke (10), die sich über den Nasenrücken eines Benutzers erstreckt; und
eine erste Backe (12) und eine zweite Backe (14), die sich von der Brücke (10) entlang jeweiliger nasaler Seitenwände der Nase des Benutzers erstrecken, sodass sie eine Komprimierungskraft anwenden, um die nasalen Seitenwände der Nase eines Benutzers zu komprimieren und den temperaturverändernden Körper in Kontakt mit den nasalen Seitenwänden zu drücken;
wobei jede Backe (12, 14) eine Außenwand (121, 141), eine Innenwand (122, 142) und ein elastisches Faltgelenk (123, 143) zwischen der Außenwand (121, 141) und der Innenwand (122, 142) umfasst, um die Innenwand (122, 142) von der Außenwand (121, 141) nach innen zu falten;
wobei die Form der Innenwand (122, 142) jeder Backe (12, 14) der Form der jeweiligen nasalen Seitenwand entspricht.

2. Nasenklemme nach einem der vorstehenden Ansprüche, wobei der Klemmkörper ein einteiliger Körper ist.

3. Nasenklemme nach einem der vorstehenden Ansprüche, wobei der Klemmkörper aus Nylon oder Polypropylen gebildet ist.

4. Nasenklemme nach einem der vorstehenden Ansprüche, wobei die Brücke (10) eine mittlere Region (101), eine erste Buckelregion (102) und eine zweite Buckelregion (103) umfasst, wobei sich die erste Buckelregion (102) und die zweite Buckelregion (103) von der mittleren Region (101) aus nach innen krümmen.

5. Nasenklemme nach einem der vorstehenden Ansprüche, ferner umfassend mindestens einen Verstärkungssteg (124), der sich zwischen der Brücke (10) und mindestens einer Backe (12, 14) erstreckt.

6. Nasenklemme nach einem der vorstehenden Ansprüche, wobei die Brücke (10) eine proximale Vorderkante (104) und eine distale Hinterkante (106) umfasst, wobei die Vorderkante (104) eine Aussparung (105) umfasst, um die Nasenspitze des Benutzers zu vermeiden, und/oder die Hinterkante (106) eine Aussparung (107) umfasst, um den Nasenbein des Benutzers zu vermeiden.

7. Nasenklemme nach einem der vorstehenden Ansprüche, wobei die Außenwände (121, 141) und Innenwände (122, 142) jeder Backe (12, 14) eine proximale Vorderkante und eine distale Hinterkante (126, 127, 146, 147) umfassen, wobei die Hinterkanten der Außenwände (126, 146) der Backe (12, 14) schräg oder gekrümmt sind, um den Gesichtskonturen eines Benutzers zu folgen.

8. Nasenklemme nach Anspruch 7, wobei die Hinterkanten der Innenwände (127, 147) der Backen (12, 14) schräg, gekrümmt und/oder gegenüber den Hinterkanten der Außenwände (126, 146) der Backen (12, 14) vertieft sind, um den Konturen der jeweiligen nasalen Seitenwände zu folgen.

9. Nasenklemme nach einem der vorstehenden Ansprüche, wobei die temperaturverändernde Vorrichtung konfiguriert ist, eine kühlende, erwärmende oder eine abwechselnde kühlende und erwärmende Wirkung bereitzustellen.

10. Nasenklemme nach einem der vorstehenden Ansprüche, wobei die temperaturverändernde Vorrichtung an einer oder beiden Innenwänden (122, 142) der Backen (12, 14) zur Platzierung gegen eine oder beide der verwendeten nasalen Seitenwände befestigt werden kann.

11. Nasenklemme nach einem der vorstehenden Ansprüche, wobei der temperaturverändernde Körper einen einteiligen Körper umfasst.

12. Nasenklemme nach einem der Ansprüche 1 bis 10, wobei der temperaturverändernde Körper ein erstes Kissen und ein zweites Kissen umfasst, die an den jeweiligen Innenwänden (122, 142) der Backen (12, 14) befestigt werden können.

13. Nasenklemme nach einem der Ansprüche 1 bis 10, wobei die temperaturverändernde Vorrichtung integral in jeder Innenwand (122, 142) des Klemmkörpers gebildet ist.

14. Satz von Teilen zur Bildung einer Nasenklemme nach einem der Ansprüche 1 bis 13, der Satz umfassend einen elastischen Klemmkörper und einen temperaturverändernden Körper, um eine temperaturverändernde Wirkung auf die Nase eines Benutzers bereitzustellen, der Klemmkörper umfassend:
eine Brücke (10), die sich über den Nasenrücken eines Benutzers erstreckt; und
eine erste Backe (12) und eine zweite Backe (14), die sich von der Brücke (10) entlang jeweiliger nasaler Seitenwände der Nase des Benutzers erstrecken, sodass sie eine Komprimierungskraft anwenden, um die nasalen Seitenwände der Nase eines Benutzers zu komprimieren und den temperaturverändernden Körper in Kontakt mit den nasalen Seitenwänden zu drücken;
wobei jede Backe (12, 14) eine Außenwand (121, 141), eine Innenwand (122, 142) und ein elastisches Faltgelenk (123, 143) zwischen der Außenwand (121, 141) und der Innenwand (122, 142) umfasst, um die Innenwand (122, 142) von der Außenwand (121, 141) nach innen zu falten;
wobei die Form der Innenwand (122,142) jeder Backe (12,14) der Form der jeweiligen nasalen Seitenwand entspricht.

## Revendications

1. Pince-nez comprenant un corps de serrage élastique et un corps de modification de température pour fournir un effet de modification de température sur le nez d'un utilisateur, le corps de serrage comprenant :
un pont (10) pour s'étendre sur le dos du nez d'un utilisateur ; et
une première mâchoire (12) et une seconde mâchoire (14) pour s'étendre à partir du pont (10) le long des parois nasales latérales respectives du nez de l'utilisateur de manière à appliquer une force de compression pour comprimer les parois nasales latérales du nez d'un utilisateur et pousser le corps de modification de température en contact avec les parois nasales latérales ;
dans lequel chaque mâchoire (12, 14) comprend une paroi externe (121, 141), une paroi interne (122, 142) et un joint de pliage élastique (123, 143) entre la paroi externe (121, 141) et la paroi interne (122, 142) pour plier la paroi interne (122, 142) vers l'intérieur à partir de la paroi externe (121, 141) ;
dans lequel la forme de la paroi interne (122, 142) de chaque mâchoire (12, 14) correspond à la forme de la paroi nasale latérale respective.

2. Pince-nez selon une quelconque revendication précédente, dans lequel le corps de serrage est un corps monobloc.

3. Pince-nez selon une quelconque revendication précédente, dans lequel le corps de serrage est formé de nylon ou de polypropylène.

4. Pince-nez selon une quelconque revendication précédente, dans lequel le pont (10) comprend une région centrale (101), une première région de voûte (102) et une seconde région de voûte (103), dans lequel la première région de voûte (102) et la seconde région de voûte (103) se courbent vers l'intérieur à partir de la région centrale (101).

5. Pince-nez selon une quelconque revendication précédente, comprenant en outre au moins une arête de renfort (124) s'étendant entre le pont (10) et au moins une mâchoire (12, 14).

6. Pince-nez selon une quelconque revendication précédente, dans lequel le pont (10) comprend un bord avant proximal (104) et un bord arrière distal (106), dans lequel le bord avant (104) comprend un évidement (105) pour éviter la pointe du nez de l'utilisateur et/ou le bord arrière (106) comprend un évidement (107) pour éviter l'os nasal du nez de l'utilisateur.

7. Pince-nez selon une quelconque revendication précédente, dans lequel les parois externes (121, 141) et les parois internes (122, 142) de chaque mâchoire (12, 14) comprennent un bord avant proximal et un bord arrière distal (126, 127, 146, 147), dans lequel les bords arrière des parois externes (126, 146) de la mâchoire (12, 14) étant inclinés ou incurvés pour suivre les contours du visage d'un utilisateur.

8. Pince-nez selon la revendication 7, dans lequel les bords arrière des parois internes (127, 147) des mâchoires (12, 14) sont inclinés, courbés et/ou en retrait par rapport aux bords arrière des parois externes (126, 146) des mâchoires (12, 14) pour suivre les contours des parois latérales nasales respectives.

9. Pince-nez selon une quelconque revendication précédente, dans lequel le dispositif de modification de température est configuré pour fournir un effet de refroidissement, de chauffage ou un effet de refroidissement et de chauffage en alternance.

10. Pince-nez selon une quelconque revendication précédente, dans lequel le dispositif de modification de température peut être fixé à l'une ou aux deux parois internes (122, 142) des mâchoires (12, 14) pour être placé contre l'une ou les deux parois nasales latérales lors de l'utilisation.

11. Pince-nez selon une quelconque revendication précédente, dans lequel le corps de modification de température comprend un corps monobloc.

12. Pince-nez selon l'une quelconque des revendications 1 à 10, dans lequel le corps de modification de température comprend un premier tampon et un second tampon pouvant être fixés aux parois internes respectives (122, 142) des mâchoires (12, 14).

13. Pince-nez selon l'une quelconque des revendications 1 à 10, dans lequel le dispositif de modification de température est formé d'un seul tenant dans chaque paroi interne (122, 142) du corps de serrage.

14. Kit de pièces pour former un pince-nez tel que défini selon l'une quelconque des revendications 1 à 13, le kit comprenant un corps de serrage élastique et un corps de modification de température pour fournir un effet de modification de température sur le nez d'un utilisateur, le corps de serrage comprenant :
un pont (10) pour s'étendre sur le dos du nez d'un utilisateur ; et
une première mâchoire (12) et une seconde mâchoire (14) pour s'étendre à partir du pont (10) le long des parois nasales latérales respectives du nez de l'utilisateur de manière à appliquer une force de compression pour comprimer les parois nasales latérales du nez d'un utilisateur et pousser le corps de modification de température en contact avec les parois nasales latérales ;
dans lequel chaque mâchoire (12, 14) comprend une paroi externe (121, 141), une paroi interne (122, 142) et un joint de pliage élastique (123, 143) entre la paroi externe (121, 141) et la paroi interne (122, 142) pour plier la paroi interne (122, 142) vers l'intérieur à partir de la paroi externe (121, 141) ;
dans lequel la forme de la paroi interne (122,142) de chaque mâchoire (12,14) correspond à la forme de la paroi nasale latérale respective.
